# EUROPEAN PATENT APPLICATION

(11) **EP 3 351 278 A1**
(43) Date of publication of application: **25.07.2018**
(21) Application number: 18153053.6
(22) Date of filing: 23.01.2018
(51) Int. Cl.: A61M 5/168, A61M 5/14

(54) **FLUID CONTROL APPARATUS**

(30) Priority: 24.01.2017 US 201762449718 P
(71) Applicant: EPIC MEDICAL PTE LTD, Singapore 079903 (SG)
(72) Inventor: LEE, Eng Hwee, Freddie, 650621 SINGAPORE (SG); WONGDEE, Whitawin, SRIRACHA (TH)
(74) Representative: Regi, François-Xavier

(57) **Abstract**

A fluid control apparatus comprises a housing; an inlet and an outlet coupled to the housing. A first flow path and a second flow path each having a front end connected to the inlet and a back end connected to the outlet to establish fluid communication between the inlet and the outlet through the first flow path and the second flow path. A flow controller is coupled to the second flow path, and a reservoir is in fluid communication with the second flow path via the flow controller. The flow controller is operable to fill fluid from the inlet to the reservoir, and to supply fluid from the reservoir to the outlet to provide a dose of supplementary medication to a patient. The apparatus may include a flow selector coupled to the first flow path to vary the flow rate of the fluid passing through the first flow path.

## Description

### Technical Field

The present invention relates to a fluid control apparatus. In particular, the present invention relates to a control apparatus for liquid medication administration.

### Background

Patient Controlled Anaesthesia (PCA) is a technology and system preconfigured by care givers according to the pain control requirements of different patients. Compared to Intramuscular injection, PCA has a better pain control effect and is capable of supplying medicine to patients when needed.

Known PCA systems and devices require close monitoring by the care givers to determine and control the actual dosage and flow rate, which results in the inconvenience and high cost to patients.

### Summary

In one embodiment, fluid control apparatus comprises a housing; an inlet and an outlet coupled to the housing. A first flow path and a second flow path each having a front end connected to the inlet and a back end connected to the outlet to establish fluid communication between the inlet and the outlet through the first flow path and the second flow path. A flow controller is coupled to the second flow path, and a reservoir is in fluid communication with the second flow path via the flow controller. The flow controller is operable to fill fluid from the inlet to the reservoir, and to supply fluid from the reservoir to the outlet to provide a dose of supplementary medication to a patient. The apparatus may include a flow selector coupled to the first flow path to vary the flow rate of the fluid passing through the first flow path.

In another embodiment, fluid control apparatus comprises a housing; an inlet and an outlet coupled to the housing. A first flow path and a second flow path each having a front end connected to the inlet and a back end connected to the outlet to establish fluid communication between the inlet and the outlet through the first flow path and the second flow path. A flow selector is coupled to the first flow path. The flow selector is switchable to allow fluid to pass from the inlet to the outlet through the first flow path.

Preferably, the first flow path further comprises a second selection conduit, a third selection conduit, a fourth selection conduit and a flow selector. The second selection conduit and the third selection conduit each has a front end connected to the inlet and a back end connected to the flow selector, the fourth selection conduit having a front end connected to the flow selector and a back end connected to the outlet.

The flow selector is switchable to a first position to close the second and the third selection conduits to allow fluid to pass from the inlet to the outlet through the first selection conduit.

Alternatively or additionally, the flow selector is switchable to open the second selection conduit, close the third selection conduit and open the fourth selection conduit to allow fluid to pass from the inlet to the outlet through the second selection conduit, the flow selector and the fourth selection conduit.

Alternatively or additionally, the flow selector is switchable to close the second selection conduit, open the third selection conduit and open the fourth selection conduit to allow fluid to pass from the inlet to outlet through the third selection conduit, the flow selector and the fourth selection conduit.

Alternatively or additionally, the flow selector is switchable to open the second, the third and the fourth selection conduits to allow fluid to pass from the inlet to the outlet through the second and the third selection conduits, the flow selector and the fourth selection conduit.

### Brief Description of drawings

The preferred embodiment of this invention is explained by the figures, by way of sample only, in which:

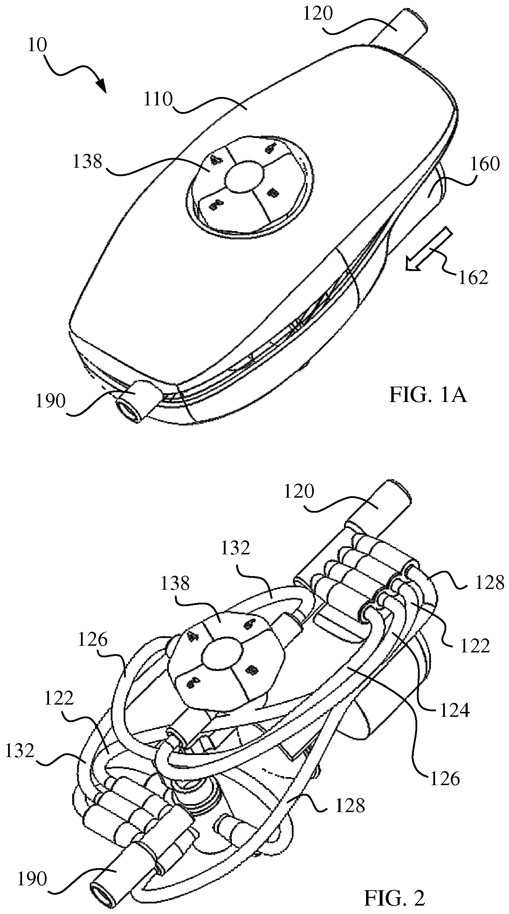

Fig. 1A is a perspective view showing a fluid control apparatus according to one embodiment of the present invention.

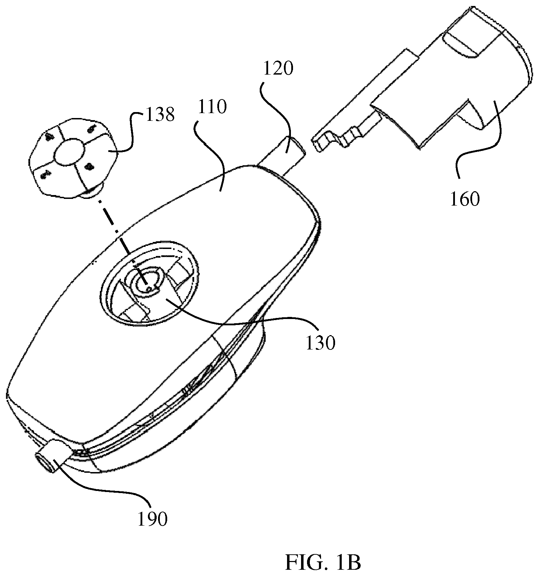

Fig. 1B is a partial exploded perspective view of Fig. 1A.
Fig. 2 is a perspective view of Fig. 1 omitting the housing to show the components in the housing.

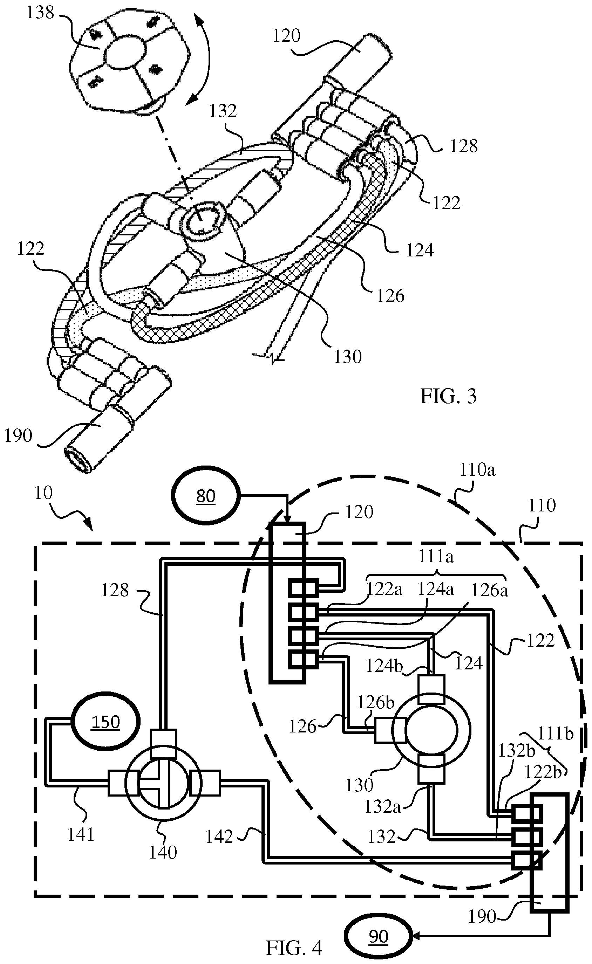

Fig. 3 is a perspective exploded view of Fig. 2 showing the first flow path and omitting the second flow path.
Fig. 4 is a schematic diagram of Fig. 2 showing the connections of the first flow path.
Fig. 5 is a perspective exploded view of Fig. 2 showing the second flow path and omitting the first flow path and before the push button is pressed.
Fig. 6 is a schematic diagram of Fig. 2 showing the connections of the second flow path and the flow controller position corresponding to Fig. 5.
Fig. 7 is a perspective exploded view of Fig. 2 showing the second flow path and omitting the first flow path and after the push button is pressed.
Fig. 8 is a schematic diagram of Fig. 2 showing the connections of the second and flow path and the flow controller position corresponding to Fig. 7.

### Detailed Description of Preferred Embodiments.

As shown in the drawings, a flow control apparatus 10 for a Patient Controlled Anaesthesia (PCA) infusion system according to one embodiment of the present invention includes a housing 110 and a fluid inlet 120 and a fluid outlet 190 coupled to the housing 110. Inlet 120 and outlet 190 may be formed integral to housing 110, or provided as separate components affixed or assembled to housing 110. A knob or dial 138 is coupled to, and rotatable relative to housing 110, for controlling and switching a flow selector 130 disposed in the housing 110, to vary the flow rate of a fluid passing through the apparatus from the inlet 120 to the outlet 190.

Inlet 120 is to be connected to a fluid source 80, for instance an infusion pump, and a fluid outlet 190 is to be connected to a patient 90 to whom a fluid such as an anaesthesia medication is to be administered. Apparatus 10 includes a first flow path 110a, in which, a front end 122a of a first selection conduit 122 is connected to inlet 120 and, a back end 122b of the first selection conduit is connected to outlet 190 to establish a fluid communication between the inlet 120 and the outlet 190, for providing a continuous, fixed flow rate of medical content from inlet 120 to outlet 190. For example, first selection conduit 122 may be sized and configured to supply a liquid medication from inlet 120 to outlet 190 under a fixed flow rate of 2ml/hour. Additionally, the first flow path 110a may include a second selection conduit 124 and a third selection conduit 126 for selectively supplying liquid medication from inlet 120 to outlet 190 under variable and predetermined flow rates, to cater to patients of different conditions and medicine administration requirements.

In one embodiment, the second selection conduit 124 and the third selection conduit 126 are connected with respective front end 124a, 126a to inlet 120, and connected with respective back end 124b, 126b to the flow selector 130. A fourth selection conduit 132 is connected with front end 132a to the flow selector 130 and with back end 132b to the outlet 190. In the context, one or more of front end 122a, 124a and 126a is/are referred to as the whole or part of the front end 111a of the first flow path 110a, and one or two of the back end 122b and 132b is/are referred to in the context as the whole or part of the back end 111b of the first flow path 110b.

Knob 138 is coupled to the flow selector 130, to enable a user e.g. a medical doctor, a nurse, a trained care giver or a patient to operate the flow selector 130, for varying the dosage and/or flow rate of the medication during administration. The flow selector 130, the knob 138 and the connections with the second selection conduit 124, third selection conduit 126 and fourth selection conduit 132 are so structured and configured such that one or both of the second selection conduit 124 and the third selection conduit 126 may be opened to supply fluid from the inlet 120 to the flow selector 130, and further from the flow selector 130 to the outlet 190 via the fourth selection conduit 132.

For example, the second selection conduit 124 is sized and configured to supply a liquid medication under a flow rate of 2ml/hour, and the third selection conduit 126 is sized and configured to supply a liquid medication under a flow rate of 4ml/hour. The knob 138 has corresponding flow rate indicators / labels shown thereon, e.g. "2, 4, 6, 8"which corresponds to flow rate of "2ml/hour", "4ml/hour", "6ml/hour" and "8ml/hour" to be supplied to the patient, respectively.

When the knob 138 is switched to a position with the label indicating "2ml/hour", the flow selector 130 closes the second selection conduit 124 and the third selection conduit 126, hence no fluid is provided from the inlet 120 to the outlet 190 via the flow selector 130, while only a fixed fluid supply of 2ml/hour flow rate takes place from the inlet 120 to the outlet 190 via the first selection conduit 122. Under this selection, a patient will receive a continuous medication administration of 2ml/hour.

When the knob 138 is switched to a position with the label indicating "4ml/hour", the flow selector 130 closes the third selection conduit 126, and meanwhile opens the second selection conduit 124 to establish fluid communication between the second selection conduit 124 and the fourth selection conduit 132, for supplying a fluid of 2ml/hour from the inlet 120 to the outlet 190 through the second selection conduit 124, the flow selector 130 and the fourth selection conduit 132, in addition to the 2ml/hour fixed flow rate provided by the first selection conduct 122. As such, under this selection, a patient will receive a continuous medication administration of 4ml/hour.

When the knob 138 is switched to a position with the label indicating "6ml/hour", the flow selector 130 closes the second selection conduit 124, and meanwhile opens the third selection conduit 126 to establish fluid communication between the third selection conduit 126 and the fourth selection fluid conduit 132, for supplying a fluid of 4ml/hour from the inlet 120 to the outlet 190 through the third selection conduit 126, the flow selector 130 and the fourth selection conduit 132, in addition to the 2ml/hour fixed flow rate provided by the first selection conduct 122. As such, under this selection, a patient will receive a continuous medication administration of 6ml/hour.

When the knob 138 is switched to a position with the label indicating "8ml/hour", the flow selector 130 opens both the second selection conduit 124 and the third selection conduit 126, to establish fluid communication between the second selection conduit 124, the third selection conduit 126 and the fourth selection conduit 132, for supplying a fluid of 2+4 = 6ml/hour from the inlet 120 to the outlet 190 through the second and the third selection conduits 124, 126 concurrently, the flow selector 140 and the fourth selection conduit 132, in addition to the 2ml/hour fixed flow rate provided by the first selection conduct 122. As such, under this selection, a patient will receive a continuous medication administration of 8ml/hour.

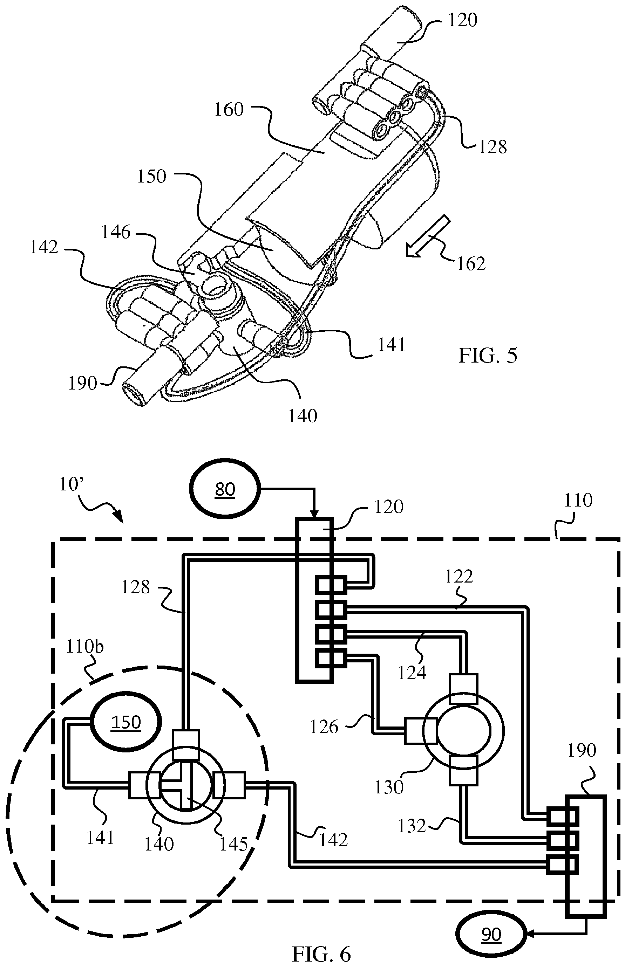

According to another aspect of the present invention, as shown in Figs. 5 to 8, an apparatus 10' may include a second flow path 110b, and a reservoir 150 for storing and supplying a supplementary bolus of liquid medication to the patient 90. Reservoir 150 is connected to a flow controller 140 via a reservoir conduit 141. A first control conduit 128 is connected between the inlet 120 and the flow controller 140. A second control conduit 142 is connected between the flow controller 140 and the outlet 190.

An actuator 146 is coupled to the flow controller140, for selectively establishing fluid connections between the first control conduit 128, the second control conduit 142 and the reservoir 150 via the flow controller140 and the reservoir conduit 141. A triggering element such as a push button 160 is slidably coupled to the housing 110, and abuts against the actuator 146 for operating the flow controller 140. A portion e.g. a finger-press portion 160a of the push button 160 may be positioned to extend out of the housing 110 to ease the control of the push button 160.

Under a default situation, push button 160 is not activated, a flow selection element 145 of the flow controller 140 is positioned (Figs. 5 and 6) to open the first control conduit 128 and close the second control conduit 142, to allow the flow controller 140 to connect the first control conduit 128 to the reservoir conduit 141, such that medication can be supplied to and fill the reservoir 150 through the first control conduit 128, the flow controller 140 and the reservoir conduit 141, from the inlet 120.

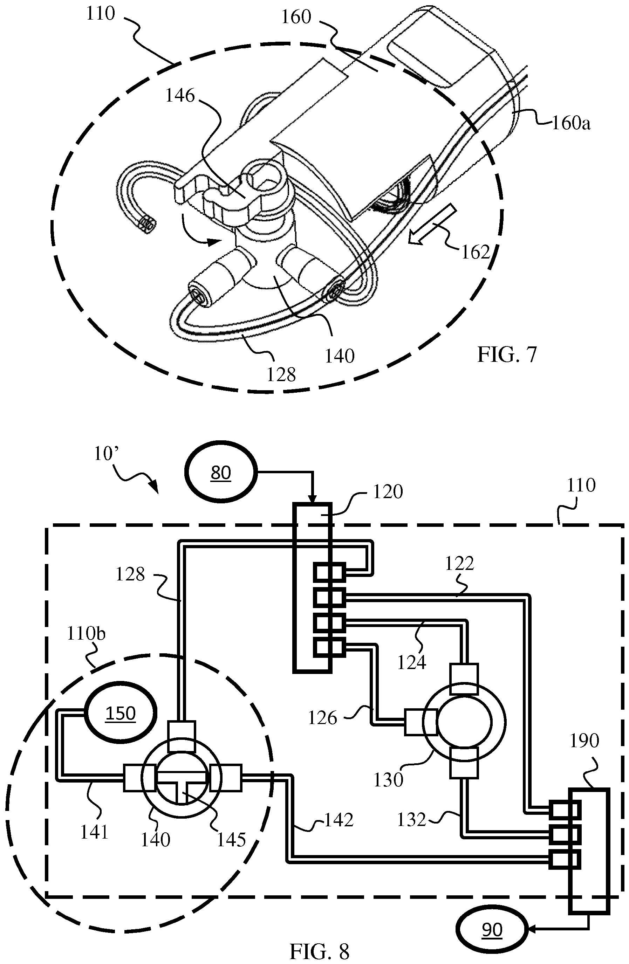

After the reservoir 150 is fully filled and when it is desired to supply a supplementary bolus of medication to the patient, the push button 160 is pressed to slide relative to the housing 110, along activation direction 162 to press against and rotate the actuator 146. As a result, the flow selection element 145 is rotated (Figs. 7 and 8) to close the first control conduit 128, and opens the second control conduit 142, to supply the medication filled in the reservoir 150 to the patient connected to the outlet 190, via the reservoir conduit 141, the flow controller 140 and the second control conduit 142.

In the present embodiment, the reservoir 150 need not be pressurised, even though the reservoir 150 may be expandable / collapsible. There is no need for fluid pressure due to action of pressing the push button 160 which operates the flow controller 140 and no need to keep pressing the reservoir when the flow controller 140 is opened. In addition, the reservoir 150 maybe merely a pliable soft container. The reservoir 150 is prevented from being pressurised when the volume space created by the rigid housing 110 is itself smaller than the fully expandable volume of the expandable reservoir.

Although embodiments of the present invention have been illustrated in conjunction with the accompanying drawings and described in the foregoing detailed description, it should be appreciated that the present invention is not limited to the embodiments disclosed. For example, it should be appreciated that the contents above disclose clearly and sufficiently, in an enabling manner, a flow control apparatus capable of varying an infusion flow rate according to predetermined infusion requirements, a flow control apparatus capable of supplying a bolus of instantaneous medication during the infusion process, and a flow control apparatus capable of both varying an infusion flow rate as well as supplying a bolus of instantaneous medication during the infusion process. Therefore, the present invention should be understood to be capable of numerous rearrangements, modifications, alternatives and substitutions without departing from the spirit of the invention as set forth and recited by the following claims.

## Claims

1. A fluid control apparatus comprising:
a housing;
an inlet and an outlet coupled to the housing;
a first flow path and a second flow path each having a front end connected to the inlet and a back end connected to the outlet to establish fluid communication between the inlet and the outlet through the first flow path and the second flow path;
a flow controller coupled to the second fluid path;
a reservoir in fluid communication with the second fluid path via the flow controller,
wherein the flow controller being operable to fill fluid from the inlet to the reservoir, and to supply fluid from the bolus reservoir to the outlet.

2. The fluid control apparatus of claim 1, further comprising a first control conduit connected between the inlet and the reservoir and a second control conduit connected between the reservoir and the outlet, wherein the flow controller being operable between a first state to open the first control conduit and close the second control conduit to fill fluid from the inlet to the reservoir, and a second state to close the first control conduit and to open the second control conduit to supply fluid from the reservoir to the outlet.

3. The fluid control apparatus of claim 2, further comprising an actuator rotatably coupled to the flow controller, the actuator being activatable to operate the flow controller from the first state to the second state.

4. The fluid control apparatus of claim 3, further comprising a triggering element coupled to the housing and abutting against the actuator, wherein the triggering element being slidable relative to the housing to activate the actuator.

5. The fluid control apparatus of claim 4, wherein the triggering element being positioned to partially extending out of the housing.

6. The fluid control apparatus of claim 1, wherein the first flow path comprises a first selection conduit having a front end connected to the inlet and a back end connected to the outlet to allow fluid to pass from the inlet to the outlet.

7. The fluid control apparatus of claim 6, wherein the first flow path further comprises a second selection conduit, a third selection conduit, a fourth selection conduit and a flow selector, wherein the second selection conduit and the third selection conduit each having a front end connected to the inlet and a back end connected to the flow selector, the fourth selection conduit having a front end connected to the flow selector and a back end connected to the outlet.

8. The fluid control apparatus of claim 7, wherein the flow selector is switchable to a first position to close the second and the third selection conduit to allow fluid to pass from the inlet to the outlet through the first selection conduit.

9. The fluid control apparatus of claim 8, wherein the flow selector is switchable to open the second selection conduit, close the third selection conduit and open the fourth selection conduit to allow fluid to pass from the inlet to the outlet through the second selection conduit, the flow selector and the fourth selection conduit.

10. The fluid control apparatus of claim 8, wherein the flow selector is switchable to close the second selection conduit, open the third selection conduit and open the fourth selection conduit to allow fluid to pass from the inlet to outlet through the third selection conduit, the flow selector and the fourth selection conduit.

11. The fluid control apparatus of claim 8, wherein the flow selector is switchable to open the second, the third and the fourth selection conduits to allow fluid to pass from the inlet to the outlet through the second and the third selection conduits, the flow selector and the fourth selection conduit.
